# EUROPEAN PATENT APPLICATION

(11) **EP 3 906 901 A1**
(43) Date of publication of application: **10.11.2021**
(21) Application number: 20382365.3
(22) Date of filing: 04.05.2020
(51) Int. Cl.: A61F 7/02

(54) **PADS AND SYSTEMS FOR TREATMENT OF A SUBJECT**

(71) Applicant: High Technology Products, SL, 08005 Barcelona (ES)
(72) Inventor: COLINA, Mónica Alejandra, 08008 Barcelona (ES); DE LOS LLANOS PÉREZ, María, 08005 Barcelona (ES); SÁNCHEZ, José Antonio, 08005 Barcelona (ES)
(74) Representative: de Rooij, Mathieu Julien

(57) **Abstract**

A pad comprising a flexible substrate having a bottom side and a top side, wherein the substrate is configured to allow heat extraction from a skin in contact with the top side of the substrate to a cooling element in contact with the bottom side, and the pad comprising a cryoprotectant gel on the top side of the substrate, and further comprising a plurality of retaining elements restricting movement of the cryoprotectant gel along the top side. Systems including such pads and a treatment device are also provided, as well as methods for treatment of subjects involving such pads.

## Description

The present disclosure relates to methods and systems for treatment a subject. The present disclosure more particularly relates to methods and systems for cosmetic treatment of a subject to locally reduce adipose tissue. The present disclosure furthermore relates to pads that may be used in such treatments. The pads may comprise a cryoprotectant to protect the skin of a subject receiving a treatment.

### BACKGROUND

Liposuction has been practiced for many years to reduce excess body fat in patients. Much less invasive treatments that aim at locally reducing adipose tissues by applying cold to a skin fold of a subject have now been offered commercially for many years as well. Subcutaneous adipose tissue has been found to be more sensitive to cold than other tissue. By applying cold to the skin, the underlying lipid-rich cells are damaged and disrupted, whereas other tissue is not damaged or less damaged. Over time, the lipid-rich cells die and disappear, through a natural apoptosis process. A cosmetic treatment for locally reducing fat can thereby be provided.

For this sort of cold treatments, treatment devices are known which have one or more applicators including a cavity. Suction may be applied to the cavity to suck in a skin fold of a subject. One or more thermally conductive (metallic) contact plates may be provided on the inside of the cavity. Thermoelectric cooling elements (Peltier elements) may be used to cool down the plates to a low temperature. Even though thermoelectric cooling elements are most widely used, alternative cooling methods based on e.g. cold fluid may be used as well.

Some form of temperature control is usually provided to control the temperature of the contact plates and thereby the temperature of the skin. In some known devices, temperature of the skin may be measured during a treatment. In other known devices instead, temperature of the conductive contact plates is measured during a treatment.

By sucking in the skin fold, improved contact between the skin and the contact plates is achieved. Additionally, by squeezing a skin fold in between contact plates, local blood flow may be reduced. Thereby supply of heat to the region can be reduced and cooling can be more effective. Applicators are also known that do not rely on suction of the skin fold.

A risk of a painful and severe injury to a patient is related to freezing of the skin or crystal formation in the skin during such a treatment. It is known that below 0.55ºC, freezing of the skin can occur depending on the time of exposure. It is known to monitor the formation of ice crystals on or inside the skin. If freezing of the skin goes unnoticed, subjects may suffer injuries related to frostbite.

In order to avoid freezing of the skin, the use of cryoprotectants is known. A cryoprotectant is a substance that can be used to protect biological tissue from freezing damage (i.e. due to large ice crystals forming).

One of the challenges related to the use of cryoprotectants in such cold treatments is related to the suction applied to the cavity in which the skin fold is introduced. The suction affects the distribution of cryoprotectant as cryoprotectant gel may be displaced to outside an area of treatment and it can accumulate at the bottom of the applicator (where an orifice through which suction is applied) is usually provided.

Another challenge is related to the effective protection of the skin by a cryoprotectant. In order for the cold treatments to be effective, the treatments may apply temperatures of less than -5ºC or less, e.g. -10ºC or less for the metallic plates during a prolonged period of time, e.g. more than 30 minutes, and more particularly more than 45 minutes and even more than one hour.

Sufficient cryoprotectant needs to be supplied to effectively protect the skin during the whole treatment.

WO 2007/127924 describes a cryoprotectant for use with a treatment device for removal of heat from subcutaneous lipid-rich cells of a subject. The cryoprotectant may be a non-freezing liquid, gel, or paste for allowing pre-cooling of the treatment device below 0ºC while preventing the formation of ice thereon. The cryoprotectant may also prevent freezing of the treatment device to the skin or ice from forming from moisture seeping out from the skin. The cryoprotectant may further be hygroscopic, thermally conductive, and biocompatible.

This prior art document discloses two different uses of a cryoprotectant. In one example, a coupling device containing the cryoprotectant, the coupling device being releasably coupled to the treatment device and proximate to the heat exchanging surface. The cryoprotectant may be replenished during a treatment. In another example, the cryoprotectant may be continually supplied using an absorbent (e.g., a cotton pad, a gauze, or other absorbents) preloaded with the cryoprotectant.

WO 2019/162539 discloses a device comprising at least one first layer, at least one second layer arranged on and joined to the first layer, defining inner surfaces between the two layers, which surfaces form at least one housing, both layers being porous and absorbent, and at least one cold-conducting anti-freezing gel, spread over the entire inner surfaces of the housing, with a freezing point below -12°C, and with a viscosity between 2,000 and 90,000 cPs at 21°C, which is sufficient to allow the gel to pass through the pores in the layers and exit the housing, wherein the device further comprises a reinforcement that acts as a stiffening structure in at least one of its layers.

It is thus known to provide an absorbent in between the metallic plates and the skin of the subject receiving a cold treatment. A cryoprotect with hygroscopic properties can attract moist or water droplets from the skin and thereby avoid freezing of the skin. Because suction is applied, one or more of the following problems may occur: the cryoprotectant liquid or gel may enter the suction system in the cavity. The suction applied to the skin can be affected and a treatment may have to be interrupted. Even if a treatment does not need to be interrupted, the cryoprotectant needs to be removed from the system after a treatment.

Another problem that may occur is that so much cryoprotectant is sucked away that no effective protection of the skin remains. A further problem that may occur is that the absorbent does not adequately liberate the cryoprotectant towards the skin, because the cryoprotectant is retained in "pockets" or pores of the absorbent.

Another problem may arise when storing absorbent pads for a prolonged period of time. These are usually stored without cryoprotectant. The absorbent pads may attract water during storage. The resulting composition of the gel used during a treatment may thus be slightly different from the foreseen cryoprotectant composition.

There still exists a need for devices which can provide for a safe and effective treatment of the skin and which avoid or reduce one or more of the aforementioned problems.

### SUMMARY

In a first aspect, a pad comprising a flexible substrate having a bottom side and a substantially non-absorbent and/or hydrophobic top side is provided, wherein the substrate is configured to allow heat extraction from a skin in contact with the top side of the substrate to a cooling element in contact with the bottom side. The pad comprises a cryoprotectant gel on the top side of the substrate, and further comprises a plurality of retaining elements restricting movement of the cryoprotectant gel along the top side.

With a pad according to this aspect, problems of retention of cryoprotectant in pockets an absorbent pad can be avoided, since the pad may be substantially hydrophobic and/or substantially non-absorbent. The retaining elements limit movement of the cryoprotectant in the plane of the pad, i.e. no barrier is provided for the movement of the gel towards the skin, but the cryoprotectant's movement towards the edges of the pad is reduced by the retaining elements. Less cryoprotectant gel than in prior art systems gets sucked into the treatment apparatus, and may even be completely avoided. This can increase safety for the subject receiving the treatment, as well as reduce maintenance of the apparatus.

Non-absorbent as used throughout the present disclosure is used to refer to a characteristic of a material or component not to absorb and retain a liquid (or gel). Substantially non-absorbent may be used throughout the present disclosure may be regarded as an absorbency of less than 10% by weight, and specifically less than 5% by weight when immersed in (demineralized) water long enough to reach saturation.

Absorbency generally is a combination of the ability of a material (or object) to capture a liquid and then to retain it. Preferably, the material used for the flexible substrate (or at least the top side thereof) may be substantially hydrophobic, and therefore not capture water or the cryoprotectant. This can also have a positive effect during storage, as the pads will not attract and retain water. The cryoprotectant used in a treatment will thus have the foreseen composition. Additionally, the top side of the flexible substrate may preferably be non-porous.

Hydrophobicity of a surface may be measured by a contact angle between droplets of water with the surface itself. Hydrophilic materials and surfaces will have a contact angle of less than 90 degrees. Hydrophobic as used throughout the present disclosure may be regarded as a surface or material having a contact angle of more than 90 degrees. In some examples, the material or substrate may be superhydrophobic, having a contact angle of between 150 and 180º.

In examples, the substrate may be made from a hydrophobic material. Examples of suitable hydrophobic materials include e.g. polyethylene (PE), polystyrene, polyvinylchloride (PVC), polytetrafluorethylene (TEFLON), polydimethylsiloxane, some polyesters, some polyurethanes, acrylics, and epoxies. In other examples, the top side of the substrate may have a specific coating to increase hydrophobicity.

In some examples, the substrate may be made from a conductive material, particularly a conductive plastic. In other examples, the substrate may be made from a material that is generally not regarded as conductive, but the substrate is made sufficiently thin so that heat extraction from the skin of the subject towards the contact plate(s) is enabled.

In some examples, the substrate may be made from a polyethylene film, and particularly may comprise LLDPE (linear low density polyethylene).

In some examples, the substrate may be made from an elastomer. Particularly, the substrate may be made from a silicone rubber with thermally conductive additives. Elastomers are flexible and easily deformable. Silicone has been used in medical applications for many years and is biocompatible. By adding thermally conductive additives, the silicone can conduct cold towards the skin (in other words, heat can be extracted from the skin).

In other examples, the substrate may be made from Nylon.

Preferably, the (silicone) substrate may be substantially non-porous. Porosity as used herein may be regarded as a fraction of the volume of voids over the total volume, as a percentage between 0% and 100%. Porosity herein may be measured through a water saturation method and may be calculated as %Pₘ= (Mₛ - M₀)/M₀ ^{∗} 100%. Herein Pₘ is the porosity or "mass porosity". Ms is the mass of an object or substance when submerged in water. M0 is the mass of the same object or substance before being submerged.

A non-porous material or substrate as used throughout the present disclosure may be regarded as a material or substrate having a porosity below 10%, specifically below1%, and more specifically as a porosity below 0.1%.

In some examples, the retaining elements may form a grid. A grid may herein be understood as a network of lines (retaining elements) that intersect each other, to form substantially rectangular or square spaces in between. One or more of the retaining elements may protrude from the top side. They may be integrally formed with the substrate, and may be made from the same material as the substrate.

In some examples, the retaining elements include a mesh attached to the top side, specifically a woven mesh, and more specifically a woven open more mesh, and even more specifically a synthetic woven open pore mesh. Such a mesh may be attached to the top side of the substrate, e.g. using an adhesive. Such a mesh may be made from e.g. polyamide (PA), polyester or polyethylene terephthalate (PET). It is preferable for the mesh to have no or little porosity such that the cryoprotectant gel is not absorbed. E.g. in some examples, the moisture absorption of the mesh at 20ºC, 65% humidity may be less than 5%, specifically less than 1%, and more specifically less than 0.5%. In some examples, the mesh may be made from a monofilament.

In some examples, a thickness of the substrate is between 5 - 1500 microns, specifically between 5 - 800 microns and more specifically between 10 and 500 microns. A thickness within this range can give the substrate (and pad) the desired flexibility and also may allow sufficient heat transfer from the skin towards the contact plates. A thickness of the substrate for less conductive substrates, e.g. silicone rubber may be e.g. between 5 and 200 microns, and more specifically between 5 and 20 microns, more specifically between 5 and 10 microns.

In some examples, the pad may furthermore comprise a sensor. The sensor may be a temperature sensor, or an impedance sensor, or other. Such a sensor may be used in the control of the treatment (e.g. temperature measurements may be used to increase or reduce cooling of the plates, or sensors may indicate the formation of ice crystals such that a treatment can be interrupted). In another example, information may be extracted from the sensor(s) to determine a correct positioning and correct use of the pad.

In a further aspect, a system is provided comprising a treatment device, and a pad according to any of the examples disclosed herein. The treatment device comprises a cavity for receiving a skin fold of a subject, one or more contact plates for contacting the skin fold, and a thermoelectric cooling system for cooling the plates, wherein the pad is configured to be arranged between the skin fold and the contact plates.

In yet a further aspect, a method for reducing adipose tissue, and particularly a cosmetic method for reducing adipose tissue is provided. The method comprises providing a treatment system including a cavity for receiving a skin fold of a subject, one or more contact plates, and a thermoelectric cooling system for cooling the contact plates; arranging a pad according to any of the examples disclosed herein on a skin fold of a subject; introducing the skin fold in the cavity of the treatment system; and cooling the skin fold during a period of up to 90 minutes.

### DESCRIPTION OF THE DRAWINGS

Non-limiting examples of the present disclosure will be described in the following, with reference to the appended drawings, in which:
Figures 1A - 1D schematically illustrate an example of a pad according to the present disclosure;
Figures 2A and 2B illustrate two alternative examples of a pad; and
Figure 3 schematically illustrates yet another example of a pad according to the present disclosure.

### DETAILED DESCRIPTION

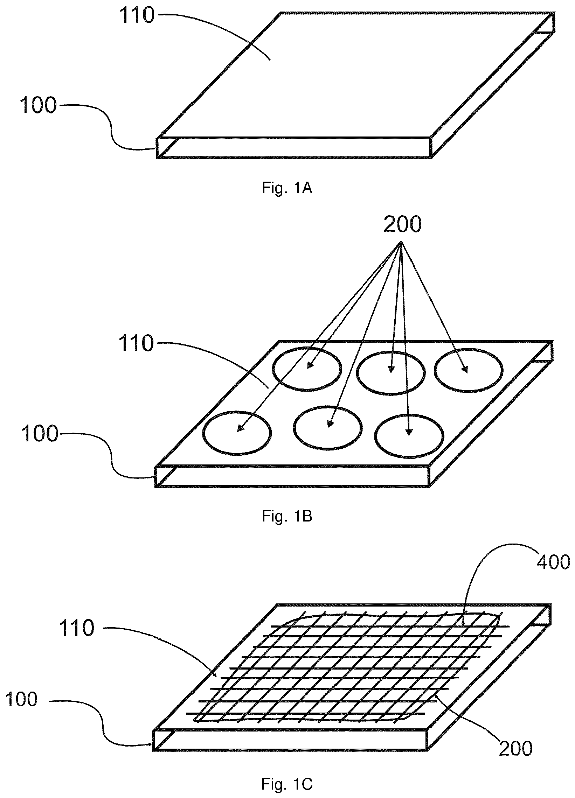

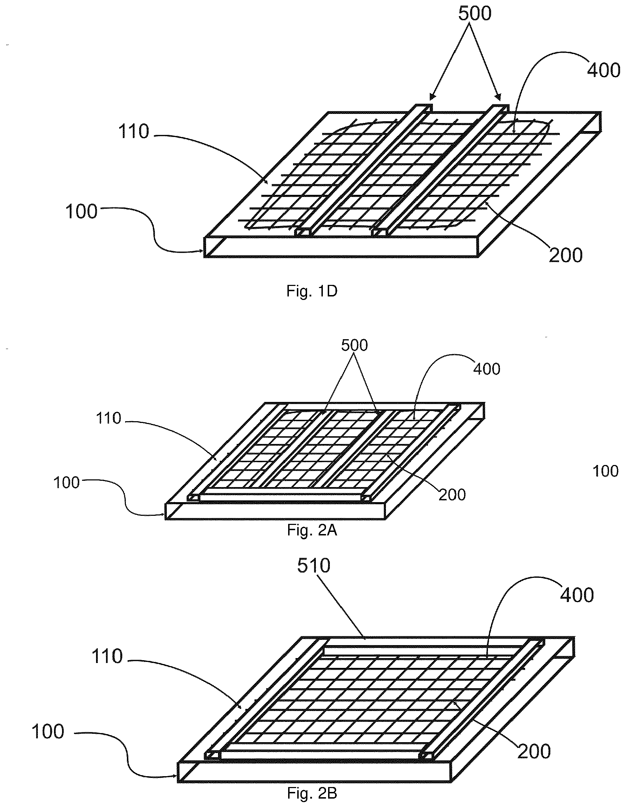

Figure 1 schematically illustrates an example of a pad according to the present disclosure. Figures 1A - 1D schematically illustrate a build-up of a pad. Such a pad may be used in a cooling treatment to reduce adipose tissue. In such a treatment, a skin fold of a subject is introduced into a cavity of an applicator of a treatment device.

Before introducing the skin fold into the cavity, a pad comprising a cryoprotectant may be positioned on the skin fold. At a bottom of the cavity one or more suction orifices may be provided. A pump may be used to suck air from the cavity and thereby suck the skin fold of the patient further into the cavity. A vacuum pump may be used to induce the suction. The induced vacuum depression is regulated by a system of electro valves that provokes vacuum and compensate for the losses that may occur.

One or more conductive plates, e.g. metallic conductive plates may be arranged within the cavity. The conductive plates may be made e.g. of aluminium or anodized aluminium. The conductive plates on one side may enter into contact with the skin of the subject, or rather with the pad positioned on top of the skin of the subject. On the opposite side, they may be in contact with Peltier elements. A Peltier cooler, or thermoelectric heat pump is a solid-state active heat pump which transfers heat from one side of the device to the other, with consumption of electrical energy, depending on the direction of the current. The heat generated by the Peltier cells may be dissipated by a heat exchanger with a circulating liquid coolant. The heat exchanger may be arranged in contact with the hot face of the Peltier cell and the coolant temperature is reduced by means of a radiator block and a fan located inside a control unit connected to the applicator.

By controlling the current (electrical power) to the Peltier elements, the temperature of the conductive plates can be controlled. Sensors, e.g. temperature sensors such as thermocouples may be used in the control of the cooling treatment.

Different applicators may be used with different treatment surfaces adapted to different areas of the body of a subject. The number of Peltier cells may be different for each applicator, and for different applicators the number of Peltier element may vary e.g. between 1 - 12 Peltier cells.

The pad according to this example comprises a flexible substrate 100 having a bottom side and a top side 110. The bottom side is configured to enter into contact with the thermally conductive plates of the treatment device. The top side 110 is configured for entering into contact with the skin of the subject.

The substrate 100 is configured to enable heat transmission from the skin (fold) of the subject to the cold conductive plates. If the substrate is very thin, e.g. in the range of 5 - 200 microns, less conductive plastics may be used. E.g. a silicone rubber substrate with a thermal conductivity of between 0.165 - 0.2 W/m.K may be used.

In some examples, the substrate may be made from a polyethylene film, and particularly may comprise LLDPE (linear low density polyethylene).

The substrate 100 may also be made of a thermally conductive plastic. The thermally conductive plastic may be an elastomer, and particularly an elastomer with thermally conductive additives. Silicone rubber filled with highly thermal conductive but electrical insulating filler zinc oxide (ZnO) fillers at various loadings may be used. Alternative additives or fillers include metal powders, graphite, aluminium oxide Al₂O₃, aluminium nitride, SiC, BN, and ZnO. Thermal conductivity of silicon may be increased to 0.5 W/m.K or more, even 0.7 W/m.K or more.

E.g. silicone rubber may be prepared with Zinc Oxide filler at 35% volume. The thermal conductivity may be more than 0.56 W/m.K. Alternatively boron nitride at 40% volume may be used as filler, and a thermal conductivity of between 0.8 and 1.4 W/m.K may be obtained.

In an alternative example, aluminium may be used as filler at 150 phr (parts per hundred rubber) for a thermal conductivity of around 0.95 W/m.K. Alternatively aluminium nitride at 150 phr may be used for a thermal conductivity of 0.7 W/m.K. And in yet a further example, 100 phr of aluminium nitride and 50 phr of alumnium may be used for an electrical conductivity of about 1 W/m.K.

A thickness of the substrate may be below 0.5 mm. The thickness may be below 0.2 or below 0.1 mm. By reducing the thickness of the substrate, heat exchange between the skin and the conductive plates can be improved.

The pad may furthermore comprise a cryoprotectant gel 200 on the top side of the substrate. The cryoprotectant 200 may include one or more of a polypropylene glycol, glycol, glycerine, polyethylene glycol, ethylene glycol, dimethyl sulfoxide, polyvinyl pyridine, calcium magnesium acetate, sodium acetate, ethanol, propanol, and sodium formate. The cryoprotectant may further comprise a thickening agent.

A preferred range of viscosity is of 10,000 - 25,000 cPs, more preferably 16,000 - 20,000 cPs at 21ºC. In some examples, a first layer with a higher viscosity, e.g. 50,000 cPs or more is provided on the substrate and a second later with a lower porosity, e.g. between 10,000 and 25,000 cPs at 21ºC may be provided. The first layer may e.g. have a porosity of up to 85,000 cPS at 21ºC, or even more.

The cryoprotectant may have a freezing point below 0ºC, preferably below -10° C, and more preferably below -20ºC.

In one example, the composition of a cryoprotectant may be as follows (in weight percentages): 45 - 50% glycerine, 42.5 - 52.5% water, 0.5 - 0.65% xanthan gum, 0.5 - 0.65% hydroxethyl cellulose.

The cryoprotectant may also have a freezing point as low as -50ºC. For example, the composition of a cryoprotectant may be (in weight percentages): 50 - 75% propylene glycol, 25 - 50% glycerine, 10 - 25% water and 0.1 - 1 % xanthan gum, 0.1 - 1% of carbomer, and less than 0.1% of sodium hydroxide.

The pad may further comprise a plurality of retaining elements 400, 500 restricting movement of the cryoprotectant gel along the top side of the substrate. In this particular example, the retaining elements include one or more meshes 400 attached to the top surface of the substrate. The mesh 400 may be a monofilament mesh and may be made from polyamide (PA) or polyethylene terephthalate (PET). In other examples, such a mesh may be metallic, and may be a monofilament metallic mesh. The mesh may be attached e.g. with tape or adhesive to the substrate.

A mesh has the effect of avoiding agglomerations of cryoprotectant gel. A further effect of the mesh is that movement of the cryoprotectant gel on the top side is reduced. As suction is applied to the cavity, the gel may have a tendency to move over the top side towards the edges of the pad and towards the suction orifice. The mesh may slow down, reduce or avoid the movement of the gel on the top surface of the pad. The mesh size and mesh opening may be such that it facilitates or promotes movement of the cryoprotectant towards the skin of the subject. In some examples, the mesh opening may be about 60% or more, specifically about 70% or more. Mesh openings in this range will liberate the cryoprotectant towards the skin during a treatment.

In a specific example, mesh size may be 300x300 microns with a filament size of 50 microns. A mesh opening of about 73% is the result in this example.

In this particular example, further retaining elements 500 are shown. These further retaining elements may be made from the same material as the substrate e.g. from silicon. They may be formed on the substrate after incorporation of the mesh. In this particular example, some of the retaining elements are therefore higher than other retaining elements.

When the cryoprotectant gel 200 is first applied to the substrate, agglomerations of cryoprotectant may occur (see figure 1B) due to the low porosity of the substrate and the surface tension of the cryoprotectant. When the mesh 400 is positioned, the cryoprotectant may be more spread over the top surface of the substrate (see figure 1C).

In some examples, the cryoprotectant on the top side of the substrate may comprise two layers with different cryoprotectants. A first layer directly on the substrate may have a higher viscosity, and a second layer on top of the first layer may have a lower viscosity. The first layer may e.g. have a viscosity of 10,000 cPs to 20,000 cPs, and the second layer may have a viscosity of more than 20,000 cPs, specifically more than 50,000 cPs at 21ºC. An aspect of having a higher viscosity for the first layer is that less cryoprotectant will move "sideways" over the substrate, whereas cryoprotectant is more easily liberated towards the skin, because of the lower viscosity for the second layer.

The substrate may be substantially rectangular. In an example, the length may be between 45 and 50 cm, and the width of the substrate may be between 25 - 35 cm. About 300 grams of cryoprotectant or more, e.g. around 400 grams may be provided on a substrate of this size.

In the example of figure 2A, further retaining elements 510 are shown which may delimit an area within which cryoprotectant gel may be provided. The retaining elements 510 may form a continuous outer barrier.

These retaining elements 510 may be arranged along and in relative vicinity of the perimeter of the pad. The retaining elements 510 may be of increased height as compared to some of the other retaining elements, and in particular in comparison with the mesh. But they may also be higher than the retaining elements 500.

Similarly, as for retaining elements 500, the retaining elements 510 along a perimeter of the pad may be made from the same material as the substrate e.g. silicone rubber. In some examples, the retaining elements 510 may be integrally formed with the substrate.

In the example of figure 2B, retaining elements 510 along a perimeter of the pad are used, in combination with a mesh. However, in the example of figure 2B, the retaining elements have not been included.

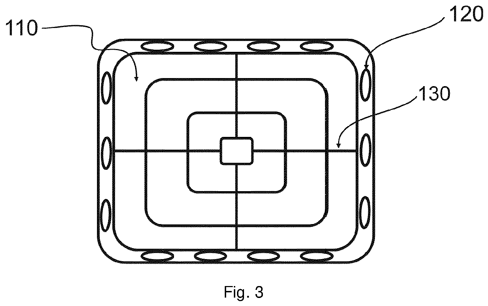

Figure 3 schematically illustrates another example of a pad according to the present disclosure. In the example of figure 2, retaining elements 130 are integrally formed with the substrate. The substrate may be made from silicone rubber, e.g. silicone rubber with thermally conductive filler.

The retaining elements 130 are made as protrusions protruding from the top side of the substrate. They may all have a similar height. The height may be between 50 - 500 microns, and specifically between 100 and 300 microns. In between the protrusions, spaces or areas are formed within which the cryoprotectant may be substantially confined. Similar as in the first example, the cryoprotectant may be liberated towards the skin but is avoided from being sucked into the treatment device.

In this particular example, the pad may comprise a plurality of suction cups 120, wherein the suction cups are arranged in proximity to and along an outer edge of the pad. The suction cups 120 may be used to secure the pad to the skin fold. The suction cups may also be integrally formed with the substrate 100. In other examples, adhesives, e.g. an adhesive tape may be used to secure the pad to the skin fold.

In any of the examples disclosed herein, the pad furthermore comprises a sensor. The sensor may be a temperature sensor, optical sensor, ultrasound sensor, or an impedance sensor, or other. The sensors may be attached to the substrate, or embedded within the substrate. Measurements from the sensor(s) may be used in the control of the cooling treatment device. I.e. temperature measurement may be used to increase or reduce cooling, e.g. by controlling a power supply to Peltier elements. An optical sensor might be used in another example to determine freezing of the skin.

Measurements of the sensor(s) may also be used to determine a correct positioning of the pad. If measurements of the sensor(s) indicate an unusual pattern, particularly at the beginning of a treatment, this may indicate that the pad is not correctly positioned and therefore that the skin of the user is not adequately protected.

In any of the examples disclosed herein, the dimensions of the pad may be adapted to the skin fold to be treated and to the dimensions of the cavity in the treatment device. A length of the pad may be between 15 and 50 cm. A width of the pad may be between 15 and 40 cm. In some examples, the length may be between 20 and 40 cm, and the width may be between 20 and 35 cm.

In any of the examples disclosed herein, a skin fold to be treated may be of one of the following areas of the subject: thigh, buttocks, abdomen, submental tissue, knees, back, face, arms. In any of the examples disclosed herein, several skin folds may be treated simultaneously. E.g. a single treatment device may comprise more than one applicator, and the applicators may be applied simultaneously to different skin folds.

In any of the examples disclosed herein, the contact plates of the treatment device may be maintained at a temperature below 0ºC, more particularly below -5ºC for a period between 15 and 90 minutes. Specially, the contact plates may be maintained in contact with the skin for a period between 20 and 75 minutes. The treatment time may be adapted to the area of skin that is being treated. Treatment times for some areas of skin may be e.g. 30 - 35 minutes, and for other areas of skin, the treatment time may be e.g. 60 - 75 minutes.

For reasons of completeness, various aspects of the present disclosure are set out in the following numbered clauses:
Clause 1. A pad comprising a flexible substrate having a bottom side and a top side which is substantially hydrophobic and/or substantially non-absorbent, wherein
   the substrate is configured to allow heat extraction from a skin in contact with the top side of the substrate to a cooling element in contact with the bottom side,
   and the pad comprises a cryoprotectant gel on the top side of the substrate, and further comprising a plurality of retaining elements restricting movement of the cryoprotectant gel along the top side.
Clause 2. The pad according to clause 1, wherein the substrate is made of a thermally conductive plastic.
Clause 3. The pad according to clause 1 or 2, wherein the substrate is made of an elastomer, particularly from silicone rubber.
Clause 4. The pad according to clause 3, wherein the substrate is made of an elastomer with thermally conductive additives, and particularly from silicone rubber with thermally conductive additives.
Clause 5. The pad according to clause 1 or 2, wherein the substrate is made of a polyolefin film material, and optionally wherein the film material is a co-extruded multilayer film.
Clause 6. The pad according to clause 5, wherein the polyolefin film material comprises polyethylene, and specifically LLDPE.
Clause 7. The pad according to any of clauses 1 - 6, wherein the retaining elements form a grid.
Clause 8. The pad according to any of clauses 1 - 7, wherein one or more of the retaining elements protrude from the top side.
Clause 9. The pad according to any of clauses 1 - 8, wherein one or more of the retaining elements are integrally formed with the substrate.
Clause 10. The pad according to any of clauses 1 - 9, wherein some of the retaining elements have an increased height compared to others of the retaining elements.
Clause 11. The pad according to clause 10, wherein the retaining elements having an increased height include one or more retaining elements along a perimeter of the pad.
Clause 12. The pad according to any of clauses 1 - 11, wherein the retaining elements include a mesh attached to the top side, specifically a woven mesh, more specifically an open pore woven mesh.
Clause 13. The pad according to clause 12, wherein the mesh is made from polyamide (PA) or polyethylene terephthalate (PET).
Clause 14. The pad according to clause 13, wherein the mesh is a metallic mesh.
Clause 15. The pad according to clause 13 or 14, wherein the mesh is a monofilament mesh.
Clause 16. The pad according to any of clauses 1 - 15, wherein a thickness of the substrate is between 5 - 1500 microns, specifically between 5 and 800 microns, and more specifically between 10 - 500 microns.
Clause 17. The pad according to any of clauses 1 - 16, further comprising one or more suction cups, wherein the suction cups are optionally arranged near an outer edge of the pad.
Clause 18. The pad according to any of clauses 1 - 17, wherein the cryoprotectant has a freezing point below -10ºC, and particularly below -20ºC.
Clause 19. The pad according to any of clauses 1 - 18, wherein a viscosity of the cryoprotectant is up to 20,000 cPs at 21ºC, specifically between 10,000 cPs and 20,000 cPs at 21ºC.
Clause 20. The pad according to any of clauses 1 - 19, including a first layer of cryoprotectant on the top side of the substrate, and a second layer of cryoprotectant on top of the first layer.
Clause 21. The pad according to clause 20, wherein a viscosity of the first layer is higher than a viscosity of the second layer.
Clause 22. The pad according to clause 20 or 21, wherein the viscosity of the second layer is less than 20,000 cPs, specifically between 10,000 cPs and 20,000 cPs at 21ºC.
Clause 23. The pad according to any of clauses 20 - 22, wherein the viscosity of the first layer is more than 20,000 cPs, specifically more than 50,000 cPs at 21ºC.
Clause 24. The pad according to any of clauses 1 - 23, wherein a length of the pad is between 15 and 50 cm, more specifically between 25 and 40 cm.
Clause 25. The pad according to any of clauses 1 - 24, wherein a width of the pad is between 15 and 40 cm, more specifically between 20 and 35 cm.
Clause 26. The pad according to any of clauses 1 - 25, wherein the pad furthermore comprises a sensor.
Clause 27. The pad according to clause 26, wherein the sensor is a temperature sensor, ultrasound sensor, optical sensor or an impedance sensor.
Clause 28. The pad according to clause 27, wherein the sensor is embedded in the substrate.
Clause 29. A system comprising a treatment device, and a pad according to any of clauses 1 - 28, the treatment device comprising
   one or more applicators, having a cavity for receiving a skin fold of a subject, and one or more contact plates for contacting the skin fold, and a thermoelectric cooling system for cooling the plates, and
   wherein the pad is configured to be arranged between the skin fold and the contact plates.
Clause 30. The system according to clause 29, wherein the bottom side of the pad is configured to enter into contact with the contact plates.
Clause 31. The system according to clause 29 or 30, wherein at least one of the applicators comprises a conductive cooling cup.
Clause 32. A method for reducing adipose tissue, the method comprising:
   providing a treatment system including a cavity for receiving a skin fold of a subject, one or more contact plates, and a thermoelectric cooling system for cooling the contact plates;
   arranging a pad according to any of clauses 1 - 28 on a skin fold of a subject;
   introducing the skin fold in the cavity of the treatment system;
   cooling the skin fold during a period of up to 90 minutes.
Clause 33. The method according to clause 32, wherein the skin fold is of one of the following areas of the subject: thigh, buttocks, abdomen, back, submental tissue, arms.
Clause 34. The method according to clause 32 or 33, wherein the contact plates are maintained at a temperature below 0ºC, more particularly below -5ºC for a period between 15 and 90 minutes, and more specifically for between 25 - 75 minutes.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow.

## Claims

1. A pad comprising a flexible substrate having a bottom side and a hydrophobic top side, wherein
the substrate is configured to allow heat extraction from a skin in contact with the top side of the substrate to a cooling element in contact with the bottom side,
and the pad comprises a cryoprotectant gel on the top side of the substrate, and further comprising a plurality of retaining elements restricting movement of the cryoprotectant gel along the top side.

2. The pad according to claim 1, wherein the substrate is made of an elastomer, particularly from silicone rubber.

3. The pad according to claim 1, wherein the substrate is made from a polyethylene film material, specifically a polyethylene film material including LLDPE.

4. The pad according to any of claims 1 - 3, wherein at least some of the retaining elements form a grid.

5. The pad according to any of claims 1 - 4, wherein one or more of the retaining elements protrude from the top side.

6. The pad according to any of claims 1 - 5, wherein one or more of the retaining elements are integrally formed with the substrate.

7. The pad according to any of claims 1 - 6, wherein some of the retaining elements have an increased height compared to others of the retaining elements.

8. The pad according to claim 7, wherein retaining elements along a perimeter of the pad have an increased height.

9. The pad according to any of claims 1 - 8, wherein the retaining elements include a mesh attached to the top side, and optionally wherein the mesh is a woven open pore mesh.

10. The pad according to claim 9, wherein the mesh is made from polyamide (PA) or polyethylene terephthalate (PET).

11. The pad according to claim 9 or 10, wherein the mesh is a monofilament mesh.

12. The pad according to any of claims 1 - 11, wherein a thickness of the substrate is between 5 - 1500 microns, specifically between 10 - 500 microns.

13. The pad according to any of claims 1 - 12, including a first layer of cryoprotectant on the top side of the substrate, and a second layer of cryoprotectant on top of the first layer, and optionally wherein the first layer of cryoprotectant has a higher viscosity than the second layer of cryoprotectant.

14. The pad according to claim 13, wherein the second layer has a viscosity of up to 20,000 cPs, specifically between 10,000 and 20,000 cPs at 21ºC, and optionally wherein the first layer has a viscosity of 50,000 cPs or higher.

15. The pad according to any of claims 1 - 14, wherein the cryoprotectant has a freezing point below -10ºC, and particularly below -20ºC.
